# EUROPEAN PATENT SPECIFICATION

(11) **EP 1 446 023 B1**
(45) Date of publication and mention of the grant of the patent: **25.02.2009**
(21) Application number: 02777492.6
(22) Date of filing: 08.11.2002
(51) Int. Cl.: A23K 1/18, A23K 1/00, A23K 1/16, A23K 1/17

(54) **FOODSTUFF FOR CATS AND DOGS**
NAHRUNGSMITTEL FÜR KATZEN UND HUNDE
ALIMENTS POUR CHATS ET CHIENS

(30) Priority: 12.11.2001 GB 0127152; 16.11.2001 GB 0127528
(43) Date of publication of application: 18.08.2004
(73) Proprietor: MARS, INCORPORATED, McLean, VA 22101-3883 (US)
(72) Inventor: GIFFARD, Catriona Julie, Wodonga, VIC 3690 (AU); KENDALL, Peter, Wodonga, VIC 3690 (AU)
(74) Representative: Cornish, Kristina Victoria Joy
(86) International application number: PCT/GB2002/005053
(87) International publication number: WO 2003/041512

(56) References cited:
- EP-A- 0 516 896
- EP-A- 0 914 831
- WO-A-94/21284
- WO-A-99/45797
- GB-A- 1 470 745
- GB-A- 1 509 339
- GB-A- 2 334 443
- US-A- 4 034 115
- US-B2- 6 312 746
- DATABASE WPI Section Ch, Week 200042 Derwent Publications Ltd., London, GB; Class B05, AN 2000-476832 XP002230393 & CN 1 247 036 A (ZHANG R), 15 March 2000 (2000-03-15)
- PATENT ABSTRACTS OF JAPAN vol. 015, no. 027 (C-0797), 22 January 1991 (1991-01-22) & JP 02 265458 A (NIKKEN FOOD KK), 30 October 1990 (1990-10-30)
- DATABASE WPI Section Ch, Week 199246 Derwent Publications Ltd., London, GB; Class D13, AN 1992-380552 XP002230394 & SU 1 704 744 A (GORKI AGRIC INST), 15 January 1992 (1992-01-15)
- DATABASE WPI Section Ch, Week 198734 Derwent Publications Ltd., London, GB; Class C03, AN 1987-239716 XP002230395 & JP 62 163662 A (NIPPON NOSAN KOGYO KK), 20 July 1987 (1987-07-20)

## Description

The present invention relates to a foodstuff, useful in the development of a healthy gastrointestinal tract.

The early development of a healthy gastrointestinal tract is key for maturation of baby animals into healthy, well protected adults. Puppies and other baby animals are born with relatively weak immune responses which means they are at greater risk of infection. They obtain natural "passive" immunity from the mother which protects them by means of antibodies that are passively transferred prior to birth *in utero.* The maternal antibody IgG travels across the placenta and provides immunity in the circulatory system of the baby to the same microbes to which their mother is immune. After birth, maternal milk provides bioactive substances and antibodies, such as IgA, that protect the digestive tract of the baby. This local passive immunity provides protection for the baby without entering the internal body cavity.

At weaning, the transition from milk-borne liquid nutrition of the mother to a solid diet is a vulnerable time for the baby. Not only does the gut have to process a solid food format but the baby is no longer able to obtain milk-borne protection from the mother. The gastrointestinal tract and immune system are still immature at weaning which means that the baby is at greater risk of infections (bacteria, viruses) that cause diarrhoea. In addition to the health risk diarrhoea reduces the growth rate of the baby and if unchecked can ultimately lead to mortality.

In addition, there are high rates of diarrhoea in puppies through the breeding and commercial sales process. Diarrhoea appears to be a common problem with pet shop owners (10-30% of puppies) and breeders (10-50% of business breeders), possibly reducing their ability to display and ultimately sell their puppy. Diarrhoea is a common reason for a visit to the vet.

EP 0194831 concerns a product for preventative and oral administration against canine parovirus containing antibodies to canine parovirus from colostrum of immunised cows. US 4034115 relates to a curdled dairy product containing probiotics. CN 1247036 relates to a nutrient that is prepared from colostrum and probiotics together with sugar and fruit juice powder. JP 02265458 relates to an anti-ageing food comprising a probiotic with colostrums and added vitamins. SU1704744 relates to a method of producing a feed for agriculture that includes colostrum and a souring agent consisting of bacteria.

Accordingly, there is a need to provide a nutritional foodstuff to help puppies, kittens and other newborn animals survive and thrive through the weaning process in particular to reduce the incidence, duration or severity of diarrhoea and to provide local passive immunity to help protect the puppy/kitten and other newborn animal as it is exposed to a series of stressful situations (leaving mother, new environment, rapid change in diet) from birth onwards. Preferably, such a nutritional foodstuff is a natural foodstuff. In addition to providing such a foodstuff, it is necessary that it is presented in a palatable form in order to encourage consumption by puppies, kittens and other baby animals.

Accordingly, the present invention provides, according to a first aspect, a foodstuff which comprises colostrum, a probiotic, a prebiotic, and a cocoa butter substitute

A probiotic is a microorganism which beneficially affects an animal host by improving its intestinal microbial balance. A prebiotic is a non-digestible food ingredient that beneficially affects the animal host by selectively promoting the growth and/or activity of one or more health promoting bacteria in the gut, thus improving host health.

Colostrum is the pre-milk fluid produced from a mother's mammary glands during the first 24-72 hours after birth. It is rich in nutrients, growth factors, immune factors and active ingredients that provide passive immunity for the young. Colostrum components include; IgA, IgG1, IgG2, IgM, IGF-1, IGF-11, TGF, EGF, lactoferrin, lysozyme, lactoperoxidase, growth hormone and insulin.

In bovine populations, bovine colostrum is the milk produced by cows for the first 1 to 4 days after the birth of the calf. It is rich in nutrients, growth factors and active ingredients that provide passive immunity for the young. The calf is able to absorb these bioactive compounds into its bloodstream for the first 24 hours of life. This is the only route for the transmission of maternal protection to the calf. The maternal antibodies, antibacterial factors and/or oligosaccharides in colostrum provide a defence against pathogenic microorganisms and viruses that can cause infection in calves. Without colostrum a significant increase in mortality rate of newborn calves occurs.

These factors in bovine colostrum can also help protect puppies, kittens and other young animals while the immune system is developing. Antibodies can bind to pathogens in the digestive tract preventing the organism from attaching to the epithelial wall of the digestive tract stopping their entry into the internal circulatory system. Thus harmful organisms are stopped before they take hold, reproduce and cause damage. In addition, the nutrients and growth factors can help ensure proper gut development, nutritional uptake and growth.

At weaning, the energy requirement of puppies, kittens and other animals is twice that of an adult of the same breed on a body weight basis. A healthy and protective gastrointestinal tract will ensure that the young animal is able to obtain maximum energy and nutrition from its food that will ensure optimal growth and development. Provision of the best nutrition during development will promote positive long term health in later life. In addition, the foodstuff of the invention provides similar health benefits to older animals.

The form or type of the foodstuff is not limiting. The form or type may depend on the animal for which the foodstuff is intended. In this respect, the foodstuff is applicable to all mammalian animals, in particular companion animals such as a pet dog (*Canis familiaris*) or a pet cat (*Felis silvestrus catus*). The foodstuff of the present invention is also applicable, to humans, in particular young humans or those in need of improving the health of their gastrointestinal tract.

The combination of a probiotic, prebiotic colostrum and a cocoa butter substitute is particularly beneficial for the entire gastrointestinal tract. Colostrum mainly benefits the stomach and the small intestine. The probiotic and the prebiotic mainly provide a benefit to the distal end of the large intestine. The combination provides a unique benefit for the entire gastrointestinal tract.

The foodstuff may be packaged. In this way, the consumer is able to identify, from the packaging, the ingredients in the food product and confirm that it is suitable for the particular animal in question.

In particular for a foodstuff for a pet dog or a pet cat, the packaging may be metal (usually in the form of a tin or flexifoil), but preferably plastic, paper or card. For a pet foodstuff, the product may be a dry, semi-moist or a moist (wet) product. Wet food includes food which is sold in tins and has a moisture content from about 70-90%. Dry food includes food having a similar composition, but with from about 5-15% moisture and presented as small biscuit-like kibbles. Semi-moist food includes food with a moisture content of between around 15% to 70% moisture.

The amount of moisture in any product may influence the type of packaging which can be used or is required. The foodstuff according to the present invention encompasses any product which the respective animal consumes in its diet. For a pet animal, the invention covers standard food products as well as pet food snacks (for example a snack bar, pet chew, crunchy treat, cereal bar, snack, biscuit and sweet product) the food product may be a cooked product. It may be in the form of a gelatinised starch matrix. It may be in the form of chunks in gravy, jelly, loaf or water. It may incorporate meat or animal derived material (such as beef, chicken, turkey, lamb, pork, fish, blood plasma, bone marrow etc or one or more thereof) the product alternatively may be meat free (optionally including a meat substitute such as soya, maize gluten or a soya product) in order to provide a protein source. The product may contain additional protein sources such as soya protein concentrate, milk proteins, gluten etc.

The product may also contain a starch source such as one or more grains (e.g. wheat, corn, rice, oats, barley etc) or may be starch free. A typical dry or semi-moist dog or cat food may contain about 20-30% crude protein and about 10-20% fat, the remainder being carbohydrate, including dietary fibre and ash. A typical wet, moist or semi-moist product may contain (on a dry matter basis) about 40% fat, 50% protein and the remainder being fibre and ash.

For a probiotic to be useful in a prepared foodstuff, it must be able to survive manufacturing processes in the preparation of the foodstuff. A commonly used process in the manufacture of a pet food, is the application of heat. The probiotic microorganism must therefore be able to survive any heating process used or must be suitable for application after any heating process has been applied.

The foodstuff of the present invention may include additional further ingredients. For example, the foodstuff may also comprise sugar. The sugar may be of any type or form, preferably having a fine grade, such as icing sugar. The benefit of a fine grade sugar is threefold. Firstly, it is a suitable carrier for the ingredients. In addition, it provides a suitable and pleasant texture and further, it reduces the water activity in the foodstuff. Reduction of water activity is a benefit in ensuring that the active components of probiotic and colostrum are protected over the shelf life of the product. Accordingly, it is a benefit that the foodstuff of the present invention is formed into a low water activity matrix. The water activity is preferably an aW of 0.6-0.1, more preferably 0.4-0.15.

A further ingredient of the foodstuff may be sorbitol. The sugar and/or sorbitol content of the foodstuff may be provided in any amount, preferably from 5-50%, more preferably from 35-45% (weight by weight per cent on a dry matter basis).

The foodstuff also includes cocoa butter or a cocoa butter substitute (or equivalent, replacer or improver). For animals which find cocoa butter palatable, then this is preferably for inclusion in the foodstuff. Such animals include humans. However, animals such as pet cats and dogs do not have a preference for cocoa butter, due to the theobromine component. Accordingly, foodstuffs which are prepared for pet animals will include a cocoa butter substitute. Cocoa butter substitutes are based on lauric fats, i.e. fats which contain a high percentage of lauric acid in their fatty acid composition. Such cocoa butter substitutes include hydrogenated oils, in particular hydrogenated vegetable oils such as hydrogenated palm kernel oil and coconut oil. Such cocoa butter or cocoa butter substitute provides good texture and is particularly useful when forming dairy like pieces of the foodstuff. One purpose of the cocoa butter/cocoa butter substitute is in the forming of moulded pieces after the mould is cooled.

The amount of the cocoa butter or cocoa butter substitute is preferably in the range of from 20-50% (weight by weight on a dry matter basis).

The foodstuff may also comprise a yoghurt component. Such a yoghurt component includes fresh or pasteurised yoghurt as well as yoghurt powder. The yoghurt component is particularly added to increase the flavour of the foodstuff. The amount of yoghurt component is preferably in the range of 1-15% (weight by weight on a dry matter basis).

In addition, one or more further ingredients may be included in the foodstuff. Examples of further ingredients may include an emulsifier, for example lecithin (0.1-2.0% weight/weight on a dry matter basis), salt (0.1-2% weight by weight on a dry matter basis), a flavour such as milk flavour (0.01-0.5% weight by weight on a dry matter basis) and/or a milk-based component, such as casein or dried milk powder.

The colostrum to be used according to the present invention is not limiting. It is preferably a highly specified colostrum and is preferably derived from bovine animals. However, the animal or origin is not limiting and other sources of colostrum include ovine or caprine sources. It may be preferable to remove or reduce some content of colostrum, such as the lactose content. Alternatively, a fraction of colostrum such as a fraction containing growth factors and/or immune components may be used. Suitable sources of colostrum include colostrum from New Live Foods, New Zealand Milk products (sold as Immulac -- a bovine colostrum powder with 15% IgG) or from NorthField (such as intact^{™}, having at least 15%IgG). In terms of safety, cows milk protein products have GRAS (Generally Regarded As Safe) status.

The present invention also encompasses fractions of colostrum which contain colostrum derived growth factors, as well as manipulated colostrum which may have an altered immunoglobulin concentration. The colostrum may be in any form, such as liquid, powdered and/or freeze-dried. The amount of colostrum (or fraction) may be in the range of 1-40% (weight by weight on a dry matter basis), as a part of the total composition or of the part of the composition comprising the colostrum.

A recommended dose of colostrum (or fraction) is approximately 0.03-0.5g/kg bodyweight per day (on a dry matter basis).

The probiotic microorganism according to the present invention is any which beneficially affects a host by improving its intestinal microbial balance. There may be one or more probiotic microorganisms present in the foodstuff. Suitable probiotic microorganisms include a considerable number of genuses, in particular *Lactobacillus* (*such as murinus, ruminus, rhamnosis, acidophilus, reuteri or mucosae*), *Bifidobacterim, Bacterioides, Aostridium, Fusobacterium, Melissococcus, Propionibacterium, Streptococcus, Enterococcus, Lactococcus, Staphylococcus, Peptostrepococcus, Bacillus, Pediococcus, Micrococcus, Leuconostoc, Weisella, Aerococcus, Oenococcus* and *Eubacterium.*

In particular, preferred probiotic microorganisms include one or more of *Lactobacillus acidophilus, Lactobacillus mucosae, Lactobacillus murinus, Lactobacillus ruminus, Lactobacillus reuteri, Bifidobacterium species* and *Bacillus subtilis.* In particular, the probiotic may be the *Lactobacillus* deposited under the Budapest Treaty on the International Recognition of the Deposit of Microorganisms for the purposes of Patent Procedure, accession number NCIMB 41117, on 10 October 2001.

The probiotic is preferably present at a level of from 10² or 10³ to 10¹⁵ cfu/g (colony forming units) per gram of the total composition or of the part of the composition comprising the probiotic. This concentration of cells provides a suitable concentration for successful colonisation of the gastrointestinal tract and providing the desired health benefit to the animal. Additional probiotics (one or more) may be present, preferably at the same concentration levels (10², 10³-10¹⁵ cfu/g of the total composition).

A recommended dose of probiotic is approximately 1x10⁸-1x10¹¹ cfu per day.

The probiotic microorganism may be in any form, for example in a powdered dry form or in spore form (for the microorganisms which form spores). In addition, the probiotic microorganism may have undergone processing in order for it to increase its survival in any processing. Accordingly, the microorganism may be coated or encapsulated in a polysaccharide, fat, starch, protein or in a sugar matrix. It may be preferable to avoid the probiotic being in contact with flour as flour contains enzymes which may adversely affect the viability of the probiotic. Standard encapsulation techniques known in the art can be used, and for example, as discussed in US 6,190,591.

The prebiotic component of the foodstuff is not limiting. It includes oligosaccharides such as glucose, fructose, xylose, galactose, lactose, mannose, arabinose, D-fucose, L-fucose, rhamnose, Actilight, Biotose, Palatinose, IMO, cellobiose, gentiobiose, laevan, maltodextrin, maltose, melibiose, raffinose, lactose, panorich, melezitose, raftiline, raftilose, stachyose, sucrose, tagatose, xylan, fructooligosaccharide (FOS), galactooligosaccharide (GOS), soy oligosaccharide, lactosucrose, maltooligosaccharide, xylooligosaccharide, inulin and fractionated inulin as well as one or more dietary fibre components such as coconut (including coconut endosperm fibre), beet pulp (such as sugarbeet pulp), chicory (including chicory pulp), oat bran concentrate, rice bran, carob bean, gum talhar and guar gum.

Oligosaccharides are naturally occurring compounds which can be found in a variety of fruits and vegetables such as bananas, tomatoes, artichokes, onions, garlic and cereals (e.g. wheat and barley). There are three varieties of FOS: 1-ketose, nystose and B-fructofuranosylnystose. While FOS can be extracted from plants such as those mentioned above, they can also be formed artificially by adding one, two or three fructose units to a sucrose molecule by a B-(2-1)-glycocosidic linkage of the fructose unit(s) to the fructose unit of sucrose. Similar artificial linking can be used to synthesise GOS and other oligosaccharides. FOS and GOS are synthetically made and sold. A single or multiple oligosaccharide may be used. One or more may be from a natural source or may be synthetic.

A single or multiple dietary fibre component may be used. A single or multiple dietary fibre component may be used in combination with a single or multiple oligosaccharide.

The form of the prebiotic according to the present invention is not limiting. It may be fresh or in any other form such as processed, heated or otherwise enzymatically treated.

The level of prebiotic incorporated into a foodstuff is not limiting. Preferably, the fibre component is present in the foodstuff at a level from approximately 0.15-8% on a dry matter basis, preferably 0.15-5% on a dry matter basis as measured by the Englyst method (as defined in English H N and Cumming J H (1984), Simplified Method for the Measurement of Total Non-Starch Polysaccharides by Gas Liquid Chromatography of Constituent Sugars as Alditol Acetates., Analyst. 109,937-942*,*). The levels, as calculated by this method, may go from 0.15% up to 5%, 6%, 7% or 8%. The lower limit may be from 1.5%, 2% or 3%. A description of the Englyst method is described in Appendix 1. The level of prebiotic may be of the total composition or of the part of the composition comprising the prebiotic.

The foodstuffs according to the present invention may be in different formats and thus the levels of colostrum, probiotic, prebiotic and a cocoa butter substitute may well differ according to the particular format.

For example, one form of foodstuff according to the present invention which is defined by the claims is a highly palatable dairy treat. The product incorporates colostrum, a prebiotic and a probiotic in a palatable delivery format. The fat-based dairy matrix is a format with low temperature processing and low water activity, optionally witch encapsulation. These elements relate to maximising the viability of the active components probiotic and colostrum. This dairy matrix can be produced as individual supplement pieces or in a larger format, more akin to a chocolate bar with delineated sections. Individual sections or pieces can then be broken off and administered to individual animals. Where the product is produced in individual supplement pieces, they can be (but are not necessarily) flow wrapped. A number of flow wrapped pieces (for example 15-25) can be placed in bottles and sealed. The bottle can have an oxygen scavenger and can be inducted sealed in order to increase the shelf life of the product. The colostrum, prebiotic and probiotic may be mixed in to any product, infused into the product or applied to the outside. Any one or more of the colostrum, prebiotic or probiotic may be in any one or more position as part of the product.

According to the present invention, a fat-based dairy matrix comprises at least 20% fat, preferably at least 30% fat. The fat may be dairy derived or from alternative non-dairy sources (such as vegetable fat).

The fat-based dairy matrix may comprise cheese or have a cheese flavour.

Suitable components (as a non-limiting example) and ranges for dairy treats with a fat-based dairy matrix are as follows:-

| Component | %wt/wt (dry matter basis) |
|---|---|
| cocoa butter substitute | 20-40 |
| sugar | 30-50 |
| colostrum (or fraction) | 5-30 |
| prebiotic | 1-5 |
| probiotic | 0.1-15 |
| emulsifier | 0.1-2 |
| salt | 0.1-2 |
| flavour (e.g. milk flavour) | 0.01-5 |
| yoghurt powder | 1-15 |

Since the format of the foodstuff may differ, the amount of individual components may vary to quite some degree. For example, a dairy treat with a fat-based dairy matrix may comprise 100% (or close to 100%) of the components above. Alternatively, the fat based dairy matrix of these components may be used as, part of another format such as a centre filling for a dry kibble. Clearly in this foodstuff format, the amount of the ingredients above will be a much smaller proportion of the total foodstuff (now a kibble).

An example of an alternative foodstuff according to the first aspect of the present invention is in the form of a dry biscuit-like kibble or cereal product. Each of the colostrum, probiotic and prebiotic may be incorporated into the product. The product may include a coating and/or filling. The coating and/or filling may contain one or more of the colostrum and/or the probiotic component. The matrix of the dried kibble or cereal product or the coating or the filling may contain the prebiotic component. Centre filled foodstuffs can be produced in accordance with standard procedures such as those described in US 3,922,353, US 3,916,029, EP 0088574, US 3,764,715, US 4,997,671 and US 6,117,477 One example of a centre filled product according to the present invention is co-extrusion of a gelatinised starch matrix and a soft centre component comprising colostrum. The end result is a small snack-like foodstuff having a soft centre which soft centre contains colostrum. The probiotic microorganism may be included in the soft centre together with the colostrum or may alternatively be incorporated in the kibble or, alternatively, applied to the outside of the kibble.

As an alternative, the soft centre may comprise a prebiotic, with the colostrum and/or probiotic being incorporated in the kibble matrix or applied to the outside thereof. This design may be preferred when the probiotic or colostrum may not survive to the manufacturing processes for the soft centre or kibble matrix.

Application of the probiotic and/or the colostrum to the outside of the matrix or other format can be carried out in a number of procedures including general coating and/or spraying. The probiotic microorganism and/or colostrum may be mixed with one or more other components when coated or sprayed to the outside of the matrix. For example, the probiotic microorganism and/or colostrum may be encapsulated or included in a lipid or protein coating.

Examples of dried, semi-moist and moist products are described in US 6,117,477 and in US 5,695,797. The present invention also provides foodstuffs according to the first aspect of the invention wherein the colostrum, probiotic and prebiotic are present when the foodstuffs are combined and eaten together. For example, the foodstuffs may comprise two or more kibble-like components of different compositions. At least one of the kibble-like biscuits comprises colostrum and at least an alternate kibble-like biscuit comprises a probiotic. Each of the kibbles may include the prebiotic component. In this foodstuff, the animal is administered each of the three components by two or more alternative sources. In addition, the colostrum or the probiotic component can be added as an alternative component to the foodstuff. For example, a kibble-like biscuit may be provided which comprises a probiotic. Such a kibble can be mixed with colostrum or prebiotic can be added in a liquid, semi-liquid or powdered format, before administration to an animal.

Alternatively a foodstuff, such as a kibble, which contains a prebiotic can have the colostrum and/or the probiotic added to it in the form of an oil, aqueous liquid or powdered topping. If the foodstuff does not contain a prebiotic, then this can also be added separately. A powder to be added can be in the form of a paper 'stick' (like sugar or instant coffee) suitably packaged to provide shelf life. The ingredients can optionally be encapsulated or coated individually, or together. Encapsulation of the ingredients permits them to travel through the alimentary canal before being subject to gut enzymes and thus may provide for a more effective and/or longer lasting result. A suitable coating is an enteric coating. Alternatively, the kibble-like biscuit may be centre filled with a colostrum-based product and the probiotic may be added as a freeze-dried component at the point of administration to the animal.

Yet a further format of the foodstuff provides the components in a powder mix. The powder mix can be added to an animal's additional food or liquid intake. The additional food may be any, such as dry kibbles, moist or semi-moist food, yoghurt, sticky snacks or treats, etc. The liquid to which the powder may be added, includes water, soup, milk, yoghurt-based drinks, milk-based drinks including chocolate or malt drinks, fruit juices, cordials, etc.

When added to the food (foodstuff or liquid) the powder can be easily consumed by the animal. The powder mix can be provided in a paper "stick" packaging or in any other manner. For example, the powder may be provided in the roof or lid of a yoghurt pot or liquid container. Suitable ranges and components for such a powder mix which is as defined by the claims are as follows, although are not limiting:-

| Component | %wt/wt (dry matter basis) |
|---|---|
| colostrum | 10-40 |
| probiotic (e.g. encapsulated) | 1-10 |
| carrying agent | 5-35 |
| milk powder (e.g. skimmed) | 20-60 |
| prebiotic | 2-20 |

The powder may contain one or more of the probiotic, prebiotic or colostrum ingredients. Preferably, it contains all three. In the mix, there may be one or more carrier materials (carrying agent) present, for example maltodextrin. The carrier is preferably dissolvable in an aqueous or fat medium. The mix may further, or alternatively, comprise one or more "free-flow" agents, such as titanium dioxide. The powder may comprise other ingredients. The powder is preferably a light colour, such as white.

Alternatively, the powder may be incorporated into a foodstuff format. For example, the powder may be in the centre of a kibble, in the centre of a snack or treat, such as with an edible coating. Such a coating may be sugar-based and/or chocolate based.

The typical proportion of any format contains around 0.1 to 10% weight by weight (on a dry matter basis) of the probiotic.

As yet a further foodstuff format, the colostrum, probiotic, prebiotic and cocoa butter substitute may be present as part of a liquid, such as water, a milk based drink, yoghurt based drink, soup, juice, etc. The probiotic is preferably part of such a liquid in an oil-based layer which sits separately. The total liquid content, when shaken, will mix, before administration.

A second aspect of the present invention provides a foodstuff according to the first aspect, for use in maintaining or improving the gastrointestinal health of an animal. All components contribute to the functional fact of the foodstuff and all in combination provide a particularly beneficial effect to the gastrointestinal health of the animal In particular, the ingredients provide the following benefits:-
- colostrum is a gentle ingredient that helps to ensure that a newborn puppy/kitten/baby's immune system has a healthy start, protecting them at this delicate time
- helps to ensure the development of a healthy digestive system optimising nutrient uptake
- helps to prevent diarrhoea and reduce the vulnerability of the animal to further episodes
- helps to protect against infection
- maintains intestinal/digestive health
- helps to improve digestion.

All features of the first aspect of the invention also apply to the second aspect

A third aspect of the invention provides a method of maintaining or improving the gastrointestinal health of an animal, the method comprising administering to the animal a foodstuff according to the first aspect of the invention.

All preferred features of the first and second aspect also apply to the third. The method according to the first aspect of the invention is particularly relevant to puppies and kittens.

A fourth aspect of the present invention provides the use of colostrum, a probiotic, a prebiotic and a cocoa butter substitute in the manufacture of a foodstuff for maintaining or improving the gastrointestinal health of an animal.

All preferred features of the first to third aspects of the invention, also apply to the fourth.

A fifth aspect of the invention provides a process for producing a foodstuff according to the first and second aspects of the invention, the process comprising mixing the raw materials optionally shaping into pieces (before or after any heating), optionally heating and presenting as a foodstuff.

The precise processing of foodstuffs may depend on the form of foodstuff.

Where no heating or cooking of the ingredients is required, the process may simply comprise mixing the raw materials for the product such as in a suitable mixer. The mixture is then spooned/scraped into moulds allowed to cool and set. The pieces are then demoulded and are ready to be packed. The bioactive components of colostrum successfully survive this process and shelf-life tests. It is preferred to store the finished product at a temperature of below 30°C to enhance shelf life.

Foodstuffs which include a type of cereal, typically require a heating/cooking step. In general, the specific processes, such as mixing, grinding, cooking, heating, extruding, or shell formation, used to make the foodstuffs are well known in the industry. Any known method can be used to form a liquid or semi-moist centre core, to form the outer shell material, to load the materials into an extruder and to co-extrude the outer and inner materials. The dried kibble-like material is usually shaped by extrusion to form pellets or kibbles. Extrusion preferably occurs at a pressure 0.2-11 MPa of (20-1000)psig and a temperature of 90-165°C.

The foodstuffs of the present invention, in differing formats, have been demonstrated to provide the probiotic in a viable, shelf-stable form.

The viability of the probiotic, in a format of a fat-based dairy matrix (such as described in Example 1) was tested. Products were stored at room temperature (in the range of 19-24°C) and samples were tested for the viability of probiotic over a 10 month period. The viability of the probiotic was excellent, with only 1 log order loss over 10 months. This level of loss is within the errors of each viability measurement.

The viability of probiotic was also tested when incorporated onto dry pet food kibble. The probiotic was coated onto the kibble in a coating base of vegetable oil and tallow. Products were stored at room temperature (in the range 19-24°C) and samples were tested for the viability of probiotic over 11 months. Viability of the probiotic was, again, excellent, with no losses over 11 months.

The present invention is further described with reference to the following drawings in which:
Figures 1a and 1b show puppy colostrum supplement pieces in two formats
Figure 2 is a general view of a kibble foodstuff of the present invention
Figure 3 is a schematic sectional view along the section line (X-X) of Figure 2 of a foodstuff of the present invention.

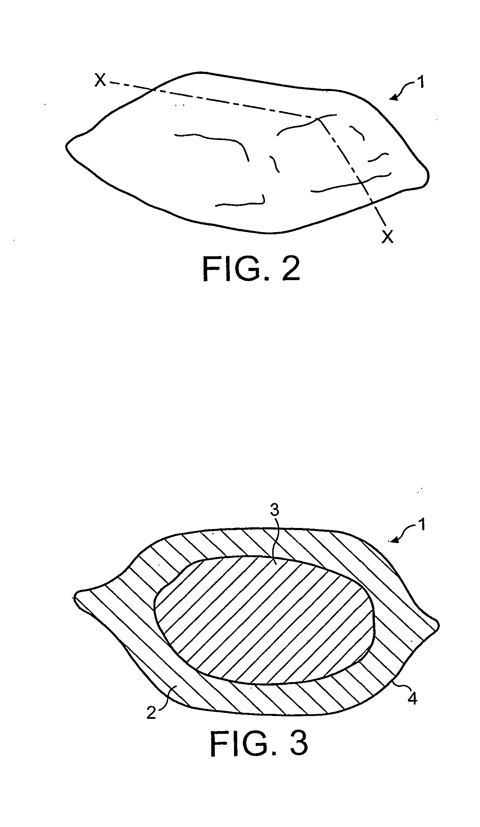

The dual texture kibble is represented as 1 in Figures 2 and 3. In Figure 3, the kibble has an outer shell 2. The outer shell (approximately 70% w/w) consists of cereals, corn, soya meal, rice and meat meal. It has a soft centre 3 which comprises a prebiotic and a coating 4 which comprises a probiotic and colostrum. The soft centre (30% w/w) consists of fats and cereals, flour, tallow and blood plasma.

The present invention is also described with reference to the following examples:

### Example 1

### Dairy Treat

A highly palatable dairy treat was produced. The recipe is as set out below:

| Recipe | |
|---|---|
| Ingredient | %wt/wt (dry matter basis) |
| hydrogenated vegetable fat | 30 |
| sucrose | 43 |
| colostrum | 15 |
| prebiotic | 3 |
| probiotic | 2 |
| emulsifier and salt | 1.6 |
| flavour | 0.4 |
| yoghurt powder | 5 |
| total | 100 |

The probiotic was present at a concentration of approximately 3 x 10¹⁰ cfu/g.

The product was obtained by mixing the raw materials. The mixture was then spooned/scraped into moulds and placed in a fridge to set. The pieces were then demoulded.

The low water activity matrix was developed using a low heat process to ensure that the active components of colostrum were protected during production and over shelf-life. Viability of this active ingredient on incorporation into the product was confined.

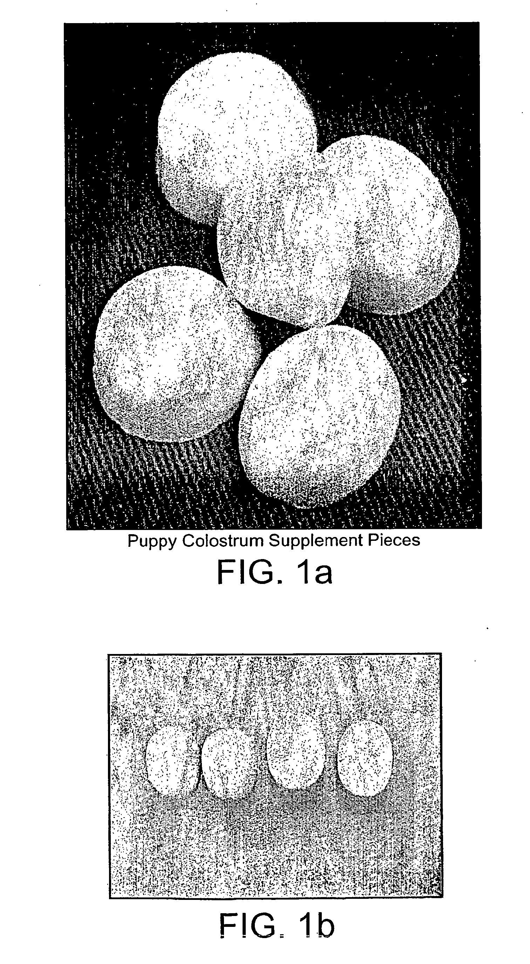

The dairy treat obtained can be seen in Figure 1.

the product is suitable for puppies and kittens from two months old through adulthood.

### Example 2

### Feeding Trial of Dairy Treat

A trial was conducted on 60 puppies. 30 of the puppies were fed a standard full and complete diet. An additional 30 puppies were fed the same diet and were also fed a dairy treat as described in Example 1 at a level of approximately 0.5g/kg bodyweight per day (a Feeding Guide for other dogs is given in Appendix 2).

All puppies were fed the basic complete diet for 2 days. Each defecation of the puppy was scored and recorded. After the 2 day wash-out period, the 30 test puppies were fed the dairy treat for a period of 10 days. The control puppies (30 in number) were continued on the complete and balanced diet for 10 days.

The results showed a significant improvement in faeces score for the puppies which were fed the dairy treat.

### Example 3

### Dual Textured Foodstuff

A dual texture kibble pet foodstuff according to the present invention was made with a shell composed of the ingredients shown in Table 1 and filled with the ingredients shown in Table 2. An outer coating was sprayed onto the shell, the content of which is shown in Table 3.

**TABLE 1**

| Ingredient | wt% |
|---|---|
| Chix w BHA/BHT | 29.00 |
| Corn whole, #2 Yellow | 39.18 |
| Rice Brewers | 17.00 |
| Soybean Meal 44% | 13.00 |
| Salt, iodized | 0.40 |
| Vitamins and Minerals | 0.80 |
| Antioxidant | 0.02 |
| Iron Oxide Colorant | 0.60 |

**TABLE 2**

| Ingredient | wt% |
|---|---|
| Hydrogenated vegetable fat | 36.2 |
| Sucrose | 45.5 |
| Colostrum | 15 |
| Inulin | 3 |
| Emulsifier | 0.3 |

**TABLE 3**

| Ingredient | wt% |
|---|---|
| *L.acidophilus* (freeze-dried) | 0.5 |
| Sunflower oil | 95.5 |

The concentration of the *L.acidophilus* used was approximately 10¹⁰ cfu/g. The approximate size of each piece is 15mm x 10mm x 5mm. The ratio (w/w) of shell:centre:coating was 3:1:0.2.

### Appendix 1

The Englyst method, from Englyst and Cummings (*Supra*).

### Experimental

### Apparatus

The fractionation procedure was carried out in 50-60ml screw-topped glass centrifuge tubes as previously described. Gas-liquid chromatography was performed with a Pye Unicam Series 204 chromatograph, fitted with a flame-ionisation detector. A 2.1m x 2mm i.d. glass column packed with Supelcoport (100-200 mesh) coated with 3% SP 2330 was used. The column temperature was 215°C (isothermal) and the injector and detector temperatures were 250°C. The carrier gas (nitrogen) flow-rate was 20ml min⁻¹.

### Reagents

High purity certified reagents were used for all analyses. Enzyme preparations were as follows: hog pancreatic α-amylase, E.C.3.2.1.1. (Sigma, Cat. No. A4268); pullulanase, E.C.3.2.1.41. (Boehringer, Cat. No. 108944).

### Method

The sequence of steps in the procedure is summarised below.

### Pre-treatment of sample

As far as possible, foods should be analysed without any pre-treatment. If there are problems in taking a representative sample, foods with a low water content can be ball milled for 2-3 minutes, and those with a higher water content homogenised, or freeze-dried and ball milled.

### Sample Mass

Accurately weigh between 50 and 1,000mg of sample, containing not more than 150mg of starch and 50mg of NSP, into a 50-60ml screw-top centrifuge tube and add a stirrer.

### Fat Extraction and Drying

Samples with dry matter between 90 and 100% and with less than 203% of fat can be analysed directly. Otherwise, add 40ml of acetone, mix for 30 minutes by using a magnetic stirrer, centrifuge and remove by aspiration as much of the supernatant as possible without disturbing the residue. Place the tubes in a water bath at 65°C on a magnetic stirrer hot plate and mix the residue for a few minutes until it appears to be dry. The beaker can be covered and the acetone vapour removed by water pump.

### Dispersion of the Starch

Add 2ml of DMSO, cap the tube and heat it in a boiling water bath for 1 hour, timed from when re-boiling commences, stirring continuously. Then, without cooling, add 8ml of 0.1M sodium acetate buffer pH5.2, at 50°C and vortex mix immediately.

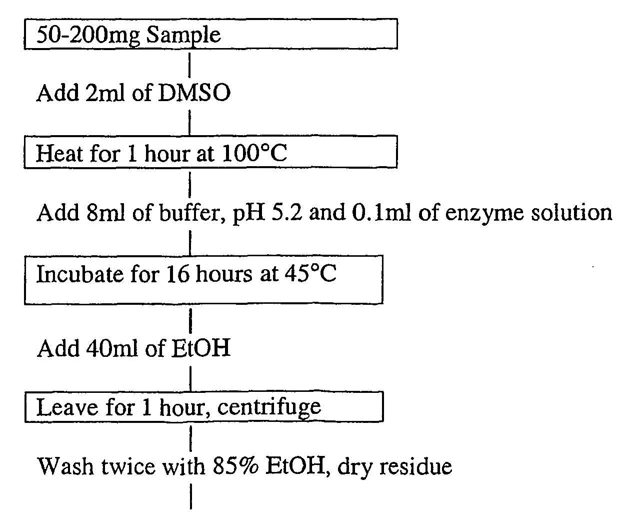

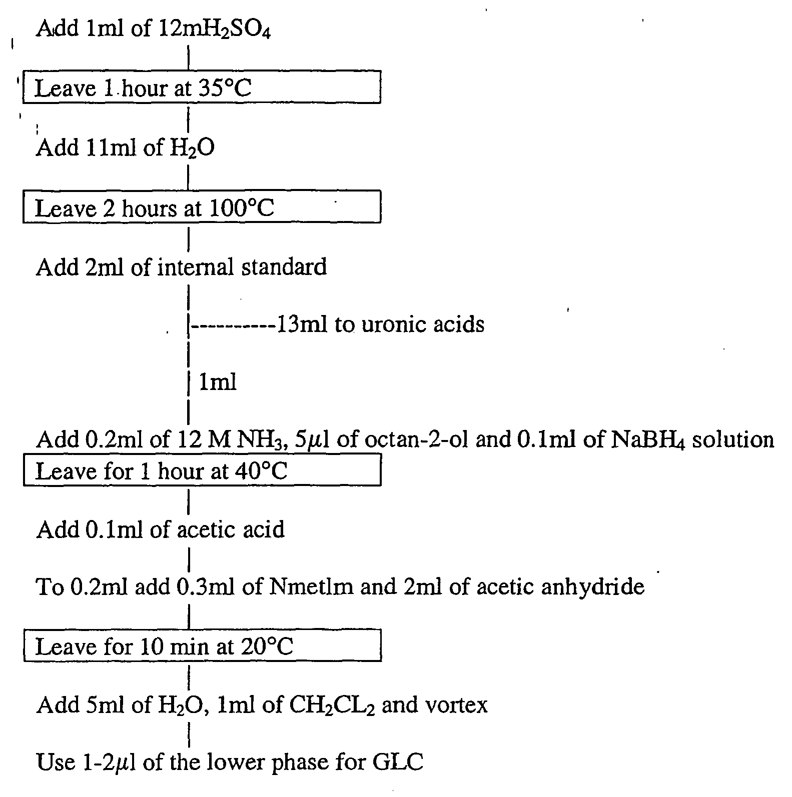

### Procedure for the analysis of non-starch polysaccharides (NSP)

### Enzyme Hydrolysis of the Starch

Cool the tube to 45°C and immediately add 0.1ml of an enzyme solution containing 5,000 units of α-amylase and 5 units of pullulanase per ml of acetate buffer at pH 5.2.
Incubate the samples at 45°C for 16-18 hours, preferably mixing continuously as described previously.

Following the enzyme treatment, add 40ml of absolute ethanol, mix well and leave to stand for 1 hour at room temperature. Centrifuge for 10 minutes or until a clear supernatant liquid is obtained. Removed by aspiration as much of the supernatant liquid as possible, without disturbing the residue, and discard it. Wash the residue twice with 50ml of 85% ethanol by mixing to form a suspension, centrifuging until clear and removing the supernatant liquid as before. Add 40ml of acetone to the washed residue, stir for 5 minutes and then centrifuge. Remove the supernatant liquid by aspiration and dry the residue as described under *Fat extraction and drying.*

### Acid hydrolysis of the residue from enzymic digestion

Disperse the dried residue in 1ml of 12M sulphuric acid, using a vortex mixer. Leave at 35°C for 1 hour to solubilise the cellulose, then rapidly add 11ml of water and mix.

Heat the solution in a boiling water bath for 2 hours from re-boiling, stirring continuously. Cool it to room temperature by placing the tube in water, add 2ml of internal standard (2 mg of allose per ml of saturated benzoic acid solution) and mix the contents of the tube. Use 1ml of the hydrolysate for the preparation of alditol acetates and keep the remainder for the determination of uronic acids.

### Uronic acids

The method used is a modification of the method of Scott. Mix 0.3ml of hydrolysate (diluted, if necessary, so that it contains between 25 and 100µg of uronic acids per ml) with 0.3ml of a mixtures of sodium chloride-boric acid solution (prepared by adding 2g of sodium chloride and 3g of boric acid to 100ml of water) Add 5ml of concentrated sulphuric acid and vortex mix, then place the tube in a heating block at 70°C. Leave the tube and contents for 40 minutes and then cool them to room temperature by placing in water. When cool, add 0.2ml of 3.5-dimethylphenol solution (0.1g of (CH₃)₂-C₆H₃OH in 100ml of glacial acetic acid) and mix immediately. Between 10 and 15 minutes later read the absorbance at 400 and 450nm in a spectrophotometer against a water reference. Subtract the reading at 400nm from that at 450nm for each sample and plot the difference obtained for glucuronic acid standards (over the range 25-125µf ml⁻¹). Read the sample concentrations from the graph.

### Preparation of alditol acetates

To 1ml of hydrolysate add 0.2ml of 12M ammonia solution and 5µl of octan-2-ol. Test that the solution is alkaline, and then add 0.1ml of a freshly prepared solution of 100mg of sodium tetrahydroborate (III) (sodium borohydride) per ml of 3M ammonia solution. Mix, leave the mixture for 1 hour at 40°C and add 0.1ml of glacial acetic acid. Next, to 0.2ml of the acidified solution add 0.3ml of N-methylimidazole and 2ml of acetic anhydride, and mix. Leave it for 10 minutes at 20°C (room temperature), add 5ml of water, mix, and when cooled add 1ml of dichloromethane, agitate the contents vigorously on a vortex mixer and centrifuge for a few minutes to separate the mixture into two phases. Remove the bulk of the upper phase by aspiration and discard it, then transfer the lower phase to a small vial, seal and store it at -20°C. Use 1-2µl for injection on to the chromatograph.

### Alternative preparative of alditol acetates

When dichloromethane is used as a solvent for the alditol acetates it has been observed in a number of laboratories without automatic GLC injection facilities that the injection technique is critical to the obtaining of reproducible results. A more robust method can be obtained if dichloromethane is replaced with ethyl acetate as a solvent for alditol acetates. The procedure is as follows:
To 1ml of hydrolysate add 0.2ml of 12M ammonia solution and 5µl of octan-2-ol. Test that the solution is alkaline, then add 0.1ml of a freshly prepared solution of 100mg of sodium tetrahydroborate (III) per ml of 3M ammonia solution. Mix, leave the mixture for 1 hour at 40°C and add 0.1ml of glacial acetic acid. To 0.5ml of the acidified solution add 0.5ml of N-methylimidazole, 5ml of acetic anhydride and mix.
Leave for 10 minutes at 20°C (room temperature), then add 0.6ml of ethanol and mix. After 5 minutes add 5ml of water, place in a water bath at room temperature, add 5ml of 7.5M KOH and a few minutes later a further 5ml of 7.5M KOH. Mix by inverting and leave to separate into two phases. Transfer the top phase to a small vial and store at +5°C. Use 1-2µl for injection on the chromatograph.

### Appendix 2

Feeding guide:

| Feeding Guide (piece per day) | | | | | | | |
|---|---|---|---|---|---|---|---|
| | BW kg | 2 mon | 3-4 mon | 5-6 mon | 7-9 mon | 10-18 mon | 19-21 mon |
| Toy | <5 | 1 | 1 | 1 | 2 | 2 | 2 |
| Small | 5 to 10 | 2 | 3 | 3 | 4 | 4 | 4 |
| Medium | 10 to 25 | 3 | 4 | 5 | 6 | 7 | 7 |
| Large | 25 to 50 | 5 | 7 | 7 | 8 | 8 | 8 |
| Giant | >50 | 5 | 7 | 8 | 8 | 8 | 8 |

| | | | | | | | |
|---|---|---|---|---|---|---|---|
| mon = months | | | | | | | |

## Claims

1. A foodstuff for a dog or a cat which comprises colostrum, a probiotic, a prebiotic and a cocoa butter substitute.

2. A foodstuff, as claimed in claim 1, further comprising sugar.

3. A foodstuff, as claimed in claim 1 or claim 2, wherein the cocoa butter substitute comprises hydrogenated vegetable oil.

4. A foodstuff, as claimed in any one of claims to 3, further comprising a yoghurt component.

5. A foodstuff, as claimed in any one of claims 1 to 4, wherein the colostrum is bovine, ovine or caprine.

6. A foodstuff, as claimed in any one of claims 1 to 5, wherein the colostrum is a fraction containing colostrum derived growth factors and immune components.

7. A foodstuff, as claimed in any one of claims 1 to 6, wherein the probiotic is one or more of *Lactobacillus, Bifidobacterium, Bacteroides, Clostridium, Fusobacterium, Melissococcus, Propionibacterium, Streptococcus, Enterococcus, Eubacterium, Lactococcus, Staphylococcus, Peptostrepococcus, Bacillus, Pediococcus, Micrococcus, Leuconostoc, Weisella, Aerococcus* and *Oenococcus.*

8. A foodstuff, as claimed in claim 7, wherein the probiotic is one or more of *Bifidobacterium species* and *Bacillus subtilis.*

9. A foodstuff, as claimed in claim 8, wherein the probiotic is one or more *Lactobacillus acidophilus,* such as the *Lactobacillus* deposited as NCIMB 41117.

10. A foodstuff, as claimed in any one of claims 1 to 9, wherein the prebiotic is one or more of glucose, fructose, xylose, galactose, lactose, mannose, arabinose, D-fucose, L-fucose, rhamnose, Actilight, Biotose, Palatinose, IMO, cellobiose, gentibiose, laevan, maltodextrin, maltose, melibiose, lactose, panorich, melezitose, raftiline, raftilose, stachyose, sucrose, tagatose, xylan, fructooligosaccharide, inulin, soy oligosaccharide, galactooligosaccharide, lactosucrose, maltooligosaccharide, xylooligosaccharide, fractionated inulin and raffinose.

11. A foodstuff, as claimed in any one of claims 1 to 10, in the form of a dried or semi-moist ready-to-eat kibble.

12. A foodstuff, as claimed in claim 11, wherein the kibble contains a coating of colostrum.

13. A foodstuff, as claimed in claim 11 or claim 12 wherein the probiotic is present on the outside of the kibble.

14. A foodstuff, as claimed in any one of claims 1 to 13 which comprises:
- a dried or moist ready-to-eat kibble comprising colostrum;
- a dried or moist ready-to-eat kibble comprising a probiotic.

15. A foodstuff, as claimed in any one of claims 1 to 13 which comprises:
- a dried or moist ready-to-eat kibble comprising a prebiotic;
- colostrum and/or probiotic in a liquid or semi-liquid format.

16. A foodstuff, as claimed in any one of claims 1 to 10, in the form of a fat-based dairy matrix.

17. A foodstuff, as claimed in any one of claims 1 to 10, in the form of a powder mix.

18. A foodstuff, as claimed in any one of claims 1 to 17, for use in maintaining or improving the gastrointestinal health of an animal.

19. Use of colostrum, a probiotic, a prebiotic and a cocoa butter substitute in the manufacture of a foodstuff for maintaining or improving the gastrointestinal health of an animal.

20. A process for producing a foodstuff, as claimed in any one of claims 1 to 18, the process comprising mixing the raw materials, optionally shaping into pieces, optionally heating and presenting as a foodstuff.

## Patentansprüche

1. Nahrungsmittel für einen Hund oder eine Katze, das Colostrum, ein Probiotikum, ein Präbiotikum und einen Kakaobutter-Ersatzstoff umfasst.

2. Nahrungsmittel nach Anspruch 1, das weiter Zucker umfasst.

3. Nahrungsmittel nach Anspruch 1 oder Anspruch 2, wobei der Kakaobutter-Ersatzstoff gehärtetes Pflanzenöl umfasst.

4. Nahrungsmittel nach einem der Ansprüche 1 bis 3, das weiter eine Joghurt-Komponente umfasst.

5. Nahrungsmittel nach einem der Ansprüche 1 bis 4, wobei das Colostrum vom Rind, vom Schaf oder von der Ziege ist.

6. Nahrungsmittel nach einem der Ansprüche 1 bis 5, wobei das Colostrum eine Fraktion ist, die aus Colostrum gewonnene Wachstumsfaktoren und Immunkomponenten enthält.

7. Nahrungsmittel nach einem der Ansprüche 1 bis 6, wobei das Probiotikum eines oder mehrere von *Lactobacillus, Bifidobacterium, Bacteroides, Clostridium, Fusobacterium, Melissococcus, Propionibacterium, Streptococcus, Enterococcus, Eubacterium, Lactococcus, Staphylococcus, Peptostrepococcus, Bacillus, Pediococcus, Micrococcus, Leuconostoc, Weisella, Aerococcus* und *Oenococcus* ist.

8. Nahrungsmittel nach Anspruch 7, wobei das Probiotikum eines oder mehrere von *Bifidobacterium species* und *Bacillus subtilis* ist.

9. Nahrungsmittel nach Anspruch 8, wobei das Probiotikum ein oder mehrere *Lactobacillus acidophilus* ist, wie etwa der *Lactobacillus,* der als NCIMB 41117 hinterlegt ist.

10. Nahrungsmittel nach einem der Ansprüche 1 bis 9, wobei das Präbiotikum eines oder mehrere von Glucose, Fructose, Xylose, Galactose, Lactose, Mannose, Arabinose, D-Fucose, L-Fucose, Rhamnose, Actilight, Biotose, Palatinose, IMO, Cellobiose, Gentibiose, Laevan, Maltodextrin, Maltose, Melibiose, Lactose, Panorich, Melezitose, Raftilin, Raftilose, Stachyose, Saccharose, Tagatose, Xylan, Fructooligosaccharid, Inulin, Sojaoligosaccharid, Galactooligosaccharid, Lactosaccharose, Maltooligosaccharid, Xylooligosaccharid, fraktioniertem Inulin und Raffinose ist.

11. Nahrungsmittel nach einem der Ansprüche 1 bis 10, in der Form eines getrockneten oder halbfeuchten verzehrfertigen Kibbles.

12. Nahrungsmittel nach Anspruch 11, wobei der Kibble eine Beschichtung aus Colostrum enthält.

13. Nahrungsmittel nach Anspruch 11 oder Anspruch 12, wobei das Probiotikum auf der Außenseite des Kibbles vorliegt.

14. Nahrungsmittel nach einem der Ansprüche 1 bis 13, welches umfasst:
- einen getrockneten oder feuchten verzehrfertigen Kibble, der Colostrum umfasst;
- einen getrockneten oder feuchten verzehrfertigen Kibble, der ein Probiotikum umfasst.

15. Nahrungsmittel nach einem der Ansprüche 1 bis 13, welches umfasst:
- einen getrockneten oder feuchten verzehrfertigen Kibble, der ein Präbiotikum umfasst;
- Colostrum und/oder Probiotikum in einem flüssigen oder halbflüssigen Format.

16. Nahrungsmittel nach einem der Ansprüche 1 bis 10, in der Form einer fettbasierten Molkereierzeugnis-Matrix.

17. Nahrungsmittel nach einem der Ansprüche 1 bis 10, in der Form eines Pulvergemisches.

18. Nahrungsmittel nach einem der Ansprüche 1 bis 17, zur Verwendung bei der Erhaltung oder Verbesserung der Magen-Darm-Gesundheit eines Tieres.

19. Verwendung von Colostrum, einem Probiotikum, einem Präbiotikum und einem Kakaobutter-Ersatzstoff bei der Herstellung eines Nahrungsmittels zur Erhaltung oder Verbesserung der Magen-Darm-Gesundheit eines Tieres.

20. Verfahren zur Herstellung eines Nahrungsmittels nach einem der Ansprüche 1 bis 18, wobei das Verfahren das Mischen der Rohmaterialien, ggf. das Ausformen zu Stücken, ggf. das Erhitzen und das Bereitstellen als ein Nahrungsmittel umfasst.

## Revendications

1. Un aliment pour chiens ou pour chats, qui contient du colostrum, un probiotique, un prébiotique et un substitut de beurre de cacao.

2. Un aliment, tel que revendiqué dans la revendication 1, comprenant en outre du sucre.

3. Un aliment, tel que revendiqué dans la revendication 1 ou la revendication 2, dans lequel le substitut de beurre de cacao comprend une huile végétale hydrogénée.

4. Un aliment, tel que revendiqué dans l'une quelconque des revendications 1 à 3, comprenant en outre composant consistant en yogourt

5. Un aliment, tel que revendiqué dans l'une quelconque des revendications 1 à 4, dans lequel le colostrum est d'origine bovine, ovine ou caprine.

6. Un aliment, tel que revendiqué dans l'une quelconque des revendications 1 à 5, dans lequel le colostrum est a fraction contenant facteurs de croissance provenant du colostrum et constituants immuns.

7. Un aliment, tel que revendiqué dans l'une quelconque des revendications 1 à 6, dans lequel le probiotique est un ou plusieurs des *Lactobacillus, Bifidobacterium, Bacteroides, Clostridium, Fusobacterium, Melissococcus, Propionibacterium, Streptococcus, Enterococcus, Eubacterium, Lactococcus, Staphylococcus, Peptostrepococcus, Bacillus, Pediococcus, Micrococcus, Leuconostoc, Weisella, Aerococcus et Oenococcus.*

8. Un aliment, tel que revendiqué dans la revendication 7, dans lequel le probiotique est un ou plusieurs *Bifidobacterium species et Bacillus subtilis.*

9. Un aliment, tel que revendiqué dans la revendication 8, dans lequel le probiotique est un ou plusieurs *Lactobacillus acidophilus,* tels que le *Lactobacillus* déposé sous le numéro NCIMB 41117.

10. Un aliment, tel que revendiqué dans l'une quelconque des revendications 1 à 9, dans lequel le prébiotique est un ou plusieurs des dérivés suivants: glucose, fructose, xylose, galactose, lactose, mannose, arabinose, Dfucose, L-fucose, rhamnose, Actilight, Biotose, Palatinose, IMO, cellobiose, gentibiose, laevane, maltodextrine, maltose, mélibiose, lactose, panorich, mélézitose, raftiline, raftilose, stachyose, sucrose, tagatose, xylane, fructooligosaccharide, inuline, oligosaccharide de soja. galactooligosaccharide; lactosucrose, maltooligosaccharide. xylooligosaccharide, inuline fractionnée et raffinose.

11. Un aliment, tel que revendiqué dans l'une quelconque des revendications 1 à 10, sous la forme d'une bouchée, sèche ou semi-humide, prête à être consommée.

12. Un aliment, tel que revendiqué dans la revendication 11, dans lequel la bouchée comporte un revêtement de colostrum.

13. Un aliment, tel que revendiqué dans la revendication 11 ou la revendication 12 dans lequel le probiotique est présent sur la face externe de la bouchée.

14. Un aliment, tel que revendiqué dans l'une quelconque des revendications 1 à 13, qui contient:
- une bouchée, sèche ou humide, prête à être consommée, comprenant du colostrum;
- une bouchée, sèche ou semi-humide, prête à être consommée, comprenant un probiotique.

15. Un aliment, tel que revendiqué dans l'une quelconque des revendications 1 à 13, qui contient:
- une bouchée, sèche ou semi-humide, prête à être consommée, comprenant un prébiotique;
- du colostrum et/ou un probiotique sous forme liquide ou semi liquide.

16. Un aliment, tel que revendiqué dans l'une quelconque des revendications 1 à 10, sous forme d'une matrice laitière grasse.

17. Un aliment, tel que revendiqué dans l'une quelconque des revendications 1 à 10, sous la forme d'un mélange pulvérulent.

18. Un aliment, tel que revendiqué dans l'une quelconque des revendications 1 à 17, destiné à être utilisé pour le maintien ou l'amélioration de l'état gastrointestinal d'un animal.

19. Utilisation de colostrum d'un probiotique, d'un prébiotique et d'un substitut de beurre de cacao pour la fabrication d'un aliment pour maintenir ou améliorer l'état gastrointestinal d'un animal.

20. Un procédé de préparation d'un aliment pour produire un aliment, tel que revendiqué dans l'une quelconque des revendications 1 à 18, ce procédé comprenant le mélange des matières premières, éventuellement leur mise en forme de pièces, éventuellement leur chauffage et leur présentation en tant qu'aliment.
